(19) **Europäisches Patentamt**
**European Patent Office**
**Office européen des brevets**

(11) **EP 2 704 091 A1**

(12) **EUROPEAN PATENT APPLICATION**
published in accordance with Art. 153(4) EPC

(43) Date of publication:
**05.03.2014 Bulletin 2014/10**

(51) Int Cl.:
*G06Q 50/10* (2012.01)       *G06Q 50/22* (2012.01)

(21) Application number: **12776263.1**

(22) Date of filing: **20.02.2012**

(86) International application number:
**PCT/JP2012/053958**

(87) International publication number:
**WO 2012/147394 (01.11.2012 Gazette 2012/44)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **25.04.2011   JP 2011097647**

(71) Applicant: **Sony Corporation**
**Tokyo 108-0075 (JP)**

(72) Inventors:
• **SHIRADO, Hirokazu**
**Tokyo 108-0075 (JP)**

• **TANAKA, Akichika**
**Tokyo 108-0075 (JP)**
• **TSUBOI, Toshimitsu**
**Tokyo 108-0075 (JP)**
• **IWAI, Yoshiaki**
**Tokyo 108-0075 (JP)**

(74) Representative: **Körber, Martin Hans**
**Mitscherlich & Partner**
**Patent- und Rechtsanwälte**
**Sonnenstrasse 33**
**80331 München (DE)**

(54) **EVALUATION DEVICE AND EVALUATION METHOD, SERVICE PROVISION SYSTEM, AND COMPUTER PROGRAM**

(57)     A service through remote control of a robot operated in an everyday living space is suitably provided.

A server of a service provider matches up a user and an operator on the basis of subjective evaluations on services received from the user and operational skill parameters received from the robot. The server selects operators to be assigned to the user for subsequent remote control services and performs scheduling of the operators to realize team building among the operators. The service provider also pays remuneration or reward to the operators.

*FIG. 1*

EP 2 704 091 A1

**Description**

TECHNICAL FIELD

The technologies disclosed herein relate to an evaluation device and an evaluation method for evaluating operators who operate a robot, a service providing system for providing services through remote control of a robot, and a computer program therefor, and in particular to an evaluation device and an evaluation method for evaluating operation of remotely controlling a robot installed in an everyday living space to provide services such as nursing care and housework assistance, a service providing system for providing services through remote control of a robot utilized in an everyday living space, and a computer program therefor.

BACKGROUND ART

As a result of rapid growth of aging society, the ratio of the aged population to the working-age population supporting them is expected to be 1 : 2.4 in 2015, and further 1 : 2.1 in 2025. There is a pressing need to create a society where the aged people, the proportion of which in the population is rapidly increasing, can enjoy active lives in health without becoming in need of nursing care as much as possible, and even those in need of nursing care can be prevented from becoming worse and live independently as much as possible.

In the future, with the increasing need of nursing care and housework assistance, the number of helpers will be insufficient if one helper is assigned to one user. There are therefore increasing demands for mechatronic devices such as robots aimed at providing housework and nursing care in place of human particularly among nursing homes for the elderly and families with the elderly.

Remote control systems in which operators remotely control robots through operator terminals have been known. For example, there is proposed a master-slave system including: a slave apparatus including a robot, a hand, a first force sensor provided on the hand, a robot controller and a hand controller; and a master apparatus having a second force sensor, and including an operation inputting/presenting device configured to receive operator force information on the basis of which operation of the slave apparatus is generated input through remote operation by an operator and present information on operation of the slave apparatus and force information from the first force sensor to the operator, and a remote control system controller configured to generate operation instruction information to the slave apparatus and the operation inputting/presenting device on the basis of information from the first force sensor and the second force sensor (refer, for example, to Patent Document 1).

It is can be conceived to provide services such as nursing care and housework assistance by using such a remote control system. Specifically, a robot can be installed in a house of a user such as a person who needs nursing care, and an operator such as a helper can remotely operate the robot to perform nursing care, housework assistance and the like. The skill of remotely controlling a robot, however, may vary among operators. There is thus a problem that the quality of services that can be provided to users is not constant even the same type of robots are installed in the houses of the users.

For example, there is an attempt to evaluate the skill of physical work of operators by using robot manipulation used in driving simulations, surgical operations and the like. With such technologies, precise evaluation can be made on physical work requiring good skills. Work in everyday spaces such as nursing care and housework assistance, however, is difficult to quantify and precise evaluation thereon cannot be kept. Furthermore, it is not sufficient to evaluate the skillfulness in operation such as operational skills on such work as nursing care and housework assistance. Regarding helpers actually engaging in nursing care and housework assistance, it is an important factor whether or not the helper is suitable for a service recipient (user) such as a person who needs nursing care.

For example, there is proposed a remote control system that matches up an operated device and an operating terminal configured to remotely operate the operated device at a server on a communication network (refer, for example, to Patent Document 2). With the remote control system, a combination of an operating terminal and an operated terminal remotely operated through the operation terminal is determined on the basis of operation executing request information at the operating side and operation executed request information at the operated side. Note that the operation executed information contains information indicating a task desired to be remotely operated and the operation executing information contains information indicating a task desired to remotely operate. There is, however, no reference to quantification of physical work, whether an operator (helper) is suitable for a service recipient (user), or team building among operators. That is, the proposed system is not sufficient as a system for remotely controlling a robot for nursing care and housework assistance.

CITATION LIST

PATENT DOCUMENTS

> Patent Document 1: Japanese Patent Application Laid-Open No. 2010-162687
> Patent Document 2: International Publication No. WO2008/140011

SUMMARY OF THE INVENTION

PROBLEMS TO BE SOLVED BY THE INVENTION

An object of the technologies disclosed herein is to provide an excellent evaluation device and an excellent evaluation method capable of suitably evaluating operators who operate a robot, an excellent service providing system capable of suitably providing services through remote control of a robot utilized in an everyday living space, and a computer program therefor.

Another object of the technologies disclosed herein is to provide an excellent evaluation device and an excellent evaluation method capable of suitably evaluating operators who operate a robot, an excellent service providing system capable of realizing matching up a user (a person who needs nursing care) and an operator (a helper) and team building among operators by combining robot manipulation and remote control services, and a computer program therefor.

SOLUTIONS TO PROBLEMS

The present application has been made in view of the aforementioned problems, and a technology described in claim 1 is an evaluation device including: a subjective evaluation acquiring unit configured to acquire a subjective evaluation made by a user on each task performed by a robot installed in the same space as the user; an operational skill parameter acquiring unit configured to acquire operational skill parameters on a plurality of operational skill items when the robot performs each task by being remotely controlled by an operator; and an analyzing unit configured to analyze a relation between the subjective evaluation acquired by the subjective evaluation acquiring unit and the operational skill parameters acquired by the operational skill parameter acquiring unit to determine an operational skill item to which the user places importance in subjective evaluation.

According to a technology described in claim 2 of the present application, the evaluation device according to claim 1 performs analysis on a plurality of operators for one user, and further includes a selecting unit configured to select an operator having a high operational skill parameter on the operational skill item to which it is determined that the user places importance in subjective evaluation for the user.

According to a technology described in claim 3 of the present application, when the operational skill parameters are continuous values or categorical variables, the analyzing unit of the evaluation device according to claim 1 is configured to perform analysis by modeling the relation between the subjective evaluation and the operational skill parameters by using multiple regression analysis.

According to a technology described in claim 4 of the present application, when the operational skill parameters are on a nominal scale, on an ordinal scale, or in three or more categories, the analyzing unit of the evaluation device according to claim 1 is configured to perform analysis by modeling the relation between the subjective evaluation and the operational skill parameters by using analysis of variance .

According to a technology described in claim 5 of the present application, when the operational skill parameters are on a nominal scale and include a few continuous values, the analyzing unit of the evaluation device according to claim 1 is configured to perform analysis by modeling the relation between the subjective evaluation and the operational skill parameters by using analysis of covariance.

According to a technology described in claim 6 of the present application, the analyzing unit of the evaluation device according to claim 1 is configured to perform analysis by modeling the relation between the subjective evaluation and the operational skill parameters by using a structural equation taking correlation between two or more operational skills into consideration.

According to a technology described in claim 7 of the present application, the operational skill parameter acquiring unit of the evaluation device according to claim 1 is configured to acquire operation time of remote control performed by an operator as an operational skill parameter.

According to a technology described in claim 8 of the present application, the operational skill parameter acquiring unit of the evaluation device according to claim 1 is configured to acquire reaction time between an instruction from the user and a reaction of the robot as an operational skill parameter.

According to a technology described in claim 9 of the present application, the operational skill parameter acquiring unit of the evaluation device according to claim 1 is configured to acquire the number of times the robot stopped during remote control as an operational skill parameter.

According to a technology described in claim 10 of the present application, the operational skill parameter acquiring unit of the evaluation device according to claim 1 is configured to acquire the number of errors in remote control performed by an operator as an operational skill parameter.

According to a technology described in claim 11 of the present application, the operational skill parameter acquiring unit of the evaluation device according to claim 1 is configured to acquire a frequency at which the robot talks to the user during operation as an operational skill parameter.

According to a technology described in claim 12 of the present application, the operational skill parameter acquiring unit of the evaluation device according to claim 1 is configured to acquire at least two of reaction time between an instruction from the user and a reaction of the robot, the number of times the robot stopped during remote control, the number of errors in remote control performed by an operator, and a frequency at which the robot talks to the user during operation as the operational skill parameters.

Furthermore, a technology described in claim 13 of the present application is an evaluation method including: a subjective evaluation acquiring step of acquiring a subjective evaluation made by a user on each task performed by a robot installed in the same space as the user; an operational skill parameter acquiring step of acquiring operational skill parameters on a plurality of operational skill items when the robot performs each task by being remotely controlled by an operator; and an analyzing step of analyzing a relation between the subjective evaluation acquired in the subjective evaluation acquiring step and the operational skill parameters acquired in the operational skill parameter acquiring step to determine an operational skill item to which the user places importance in subjective evaluation.

The present application has been made in view of the aforementioned problems, and a technology described in claim 14 is a service providing system including: a robot installed in the same space as a user; an operator terminal through which an operator remotely controls the robot; and a service provider server configured to match up a user with an operator on a basis of a subjective evaluation made by the user on each task performed by the robot and results of measuring operational skill parameters when the robot performs each task by being remotely controlled by the operator.

A "system" used herein refers to a logical group of devices (or functional modules realizing certain functions) regardless of whether or not the devices or the functional modules are within a single housing.

Furthermore, a technology described in claim 15 is a computer program described in a computer-readable format and causing a computer to function as: a subjective evaluation acquiring unit configured to acquire a subjective evaluation made by a user on each task performed by a robot installed in the same space as the user; an operational skill parameter acquiring unit configured to acquire operational skill parameters on a plurality of operational skill items when the robot performs each task by being remotely controlled by an operator; and an analyzing unit configured to analyze a relation between the subjective evaluation acquired by the subjective evaluation acquiring unit and the operational skill parameters acquired by the operational skill parameter acquiring unit to determine an operational skill item to which the user places importance in subjective evaluation.

The computer program according to claim 15 of the present application defines a computer program described in a computer-readable format so as to realize predetermined processing on a computer. In other words, as a result of installing the computer program according to claim 15 of the present application in a computer, cooperative operations are performed on the computer and the operations and effects similar to those of the evaluation device according to claim 1 of the present application can be produced.

EFFECTS OF THE INVENTION

According to the technologies disclosed herein, an excellent evaluation device and an excellent evaluation method capable of suitably evaluating operators who operate a robot, an excellent service providing system capable of realizing matching up a user (a person who needs help) and an operator (a helper) and teambuilding among operators by combining robot manipulation and remote control services, and a computer program therefor can be provided.

Other objects, features, and advantages of the technologies disclosed herein will be apparent from more detailed description based on embodiments and accompanying drawings provided below.

BRIEF DESCRIPTION OF DRAWINGS

Fig. 1 is a diagram showing an example structure of a service providing system according to an embodiment of the technologies disclosed herein.
Fig. 2 is a diagram schematically showing a flow of system operation in the service providing system shown in Fig. 1.

Fig. 3 is a diagram showing examples of subjective evaluations from a user and operational skill parameters that are compiled.

Fig. 4 is a table showing examples of subjective evaluations from a user and operational skill parameters that are compiled.

MODES FOR CARRYING OUT THE INVENTION

Embodiments of the technologies disclosed herein will be described in detail below with reference to the drawings.

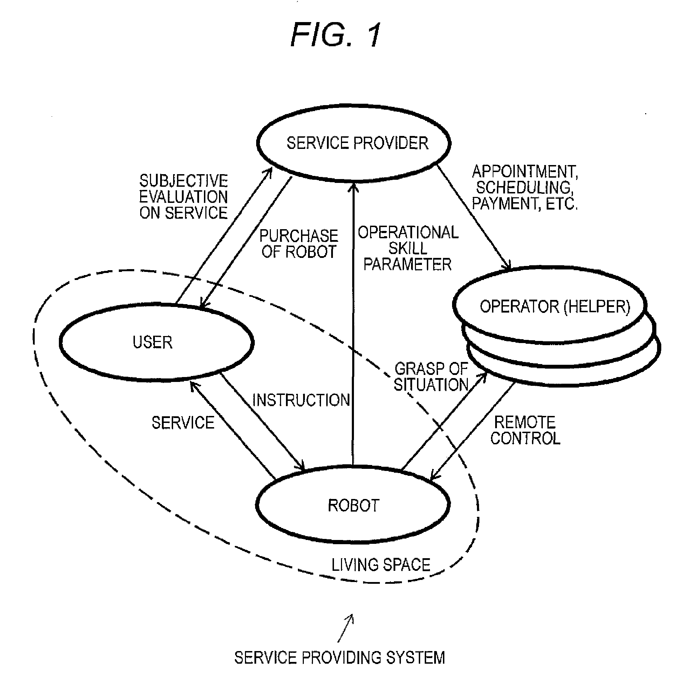

Fig. 1 schematically shows an example structure of a service providing system according to an embodiment of the technologies disclosed herein. The shown system includes four types of members including a service provider, one or more users, a plurality of operators, and a robot, and is configured to provide services through remote control of a robot utilized in an everyday living space. Specifically, the services provided in an everyday living space through remote operation of a robot refer to nursing care, housework assistance, and the like a person who needs nursing care. Accordingly, a user corresponds to a person who needs nursing care, and each operator who remotely operates a robot corresponds to a helper. The user (more specifically, a user terminal of the user), the robot and the operators (more specifically, operator terminals for remotely controlling the robot) are each connected to the service provider (more specifically, a server operated by the service provider). Furthermore, the robot purchased by the user and the operator terminals of operators who remotely operate the robot are interconnected via a transmission medium such as a network.

When the user purchase a robot from the service provider, the robot is installed in the living space of the user. Then, when the user gives an instruction to the robot, the robot transmits the instruction from the user in the form of video information, audio information or other information to an operator. The operator grasps the situation in the living space of the user on the basis of the transmitted information, and remotely operates the robot through the operator terminal. In this manner, the robot provides services such as nursing care and housework assistance in the living space to the user.

The robot realizes operation such as carrying an object as a result of being remotely controlled, and at the same time quantifies the skill of the remote control performed by the operator and transmits the quantification result as an operational skill parameter to the server of the service provider. In the meantime, the user, who has received a service from the robot, transmits a subjective evaluation on the service through the user terminal to the server of the service provider.

The server of the service provider matches up the user and an operator on the basis of subjective evaluations on services received from the user and operational skills of operators quantitatively evaluated through the robot that is remotely controlled, that is, operational skill parameters. The server then selects operators to be assigned to the user for subsequent remote control services and performs scheduling of the operators to realize team building among the operators. The service provider also pays remuneration or reward for engaging in remote control services to the operators.

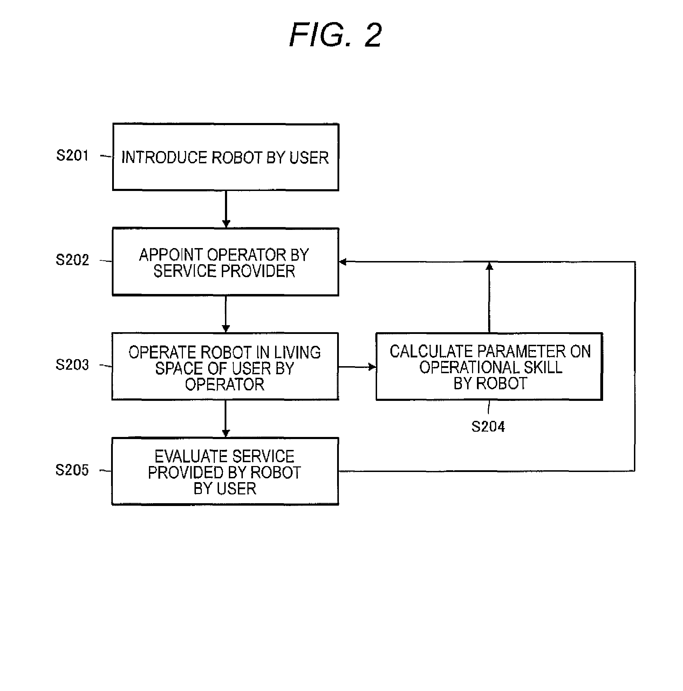

Fig. 2 schematically shows a flow of system operation in the service providing system shown in Fig. 1.

First, when the user purchases a robot to be remotely controlled from the service provider, the robot is introduced in the living space of the user himself/herself (step S201).

Following the introduction of the robot into the living space, the service provider who provided the robot appoints operators who remotely operate the robot (step S202). In this case, an operator alone or a team of operators supports one user.

As described above, when the user gives an instruction to the robot in the living space, the robot transmits the instruction in the form of video information, audio information or other information to an operator. In response thereto, the operator grasps the situation in the living space of the user on the basis of the transmitted information and remotely operates the robot through the operator terminal. The robot being remotely operated performs a task such as carrying an object, clearance, watch over the user, or guiding the user to provide the user with a service such as nursing care and housework (step S203).

While providing a service such as moving an object by being remotely controlled through the operator terminal, the robot quantitatively evaluates the operational skill of the operator and calculates the evaluation as an operational skill parameter (step S204). Examples of operational skill parameters that are calculated include the operation time of a service (remote control), the reaction time of the robot between an instruction from the user and the reaction of the robot, the number of times the robot stopped during remote control performed by the user, the number of errors in remote control performed by the user, and the frequency at which the robot talks to the user during operation, and these values are measured each time a task is performed by the robot.

In the meantime, the user gives instructions to the operators through the robot and receives services such as nursing care and housework assistance. Then, the user who has received a service can inform the server of the service provider of a subjective evaluation on the service through the user terminal (step S205). The subjective evaluation from the user is a score given subjectively by the user to each task performed by the robot.

The provider of the services compiles the subjective evaluations from the user and the operational skill parameters acquired from the robot. The provider of the services can then use the compiled data for evaluation of the operators, changing operators, appointment of operators for a new user as described above, and team building among operators appropriate for services required by the user.

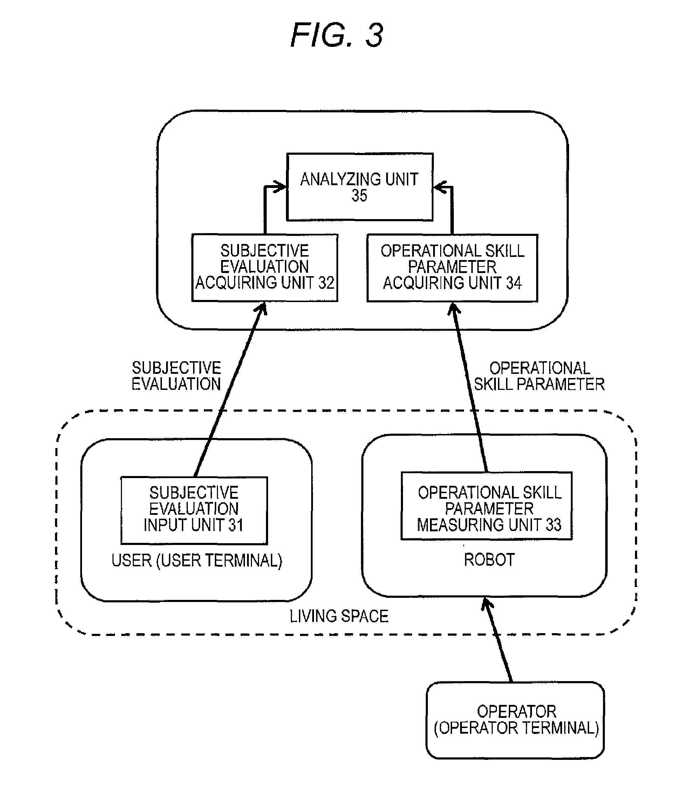

Fig. 3 show a functional structure for realizing appointment of operators for a user who purchased a robot and team building among operators appropriate for services required by the user in the service providing system shown in Fig. 1.

When the robot installed in the living space of the user performs a task, the user inputs a subjective evaluation on the task to a subjective evaluation input unit 31 of a user terminal or the like, for example. At the server of the service provider, a subjective evaluation acquiring unit 32 then acquires the subjective evaluation input by each user.

The robot installed in the living space of the user also performs a task in response to remote control performed by an operator using an operator terminal. The robot also operates as an operational skill parameter measuring unit 33 to measure operational skill parameters on multiple operational skill items when each task is performed. At the server of the service provider, an operational skill parameter acquiring unit 34 then acquires the operational skill parameters on the operational skill items measured by the operational skill parameter measuring unit 33.

An analyzing unit 35 in the server of the service provider analyzes the relation between the subjective evaluations acquired by the subjective evaluation acquiring unit 32 and the operational skill parameters acquired by the operational skill parameters acquiring unit 34, and determines an operational skill item to which the user places importance in subjective evaluation.

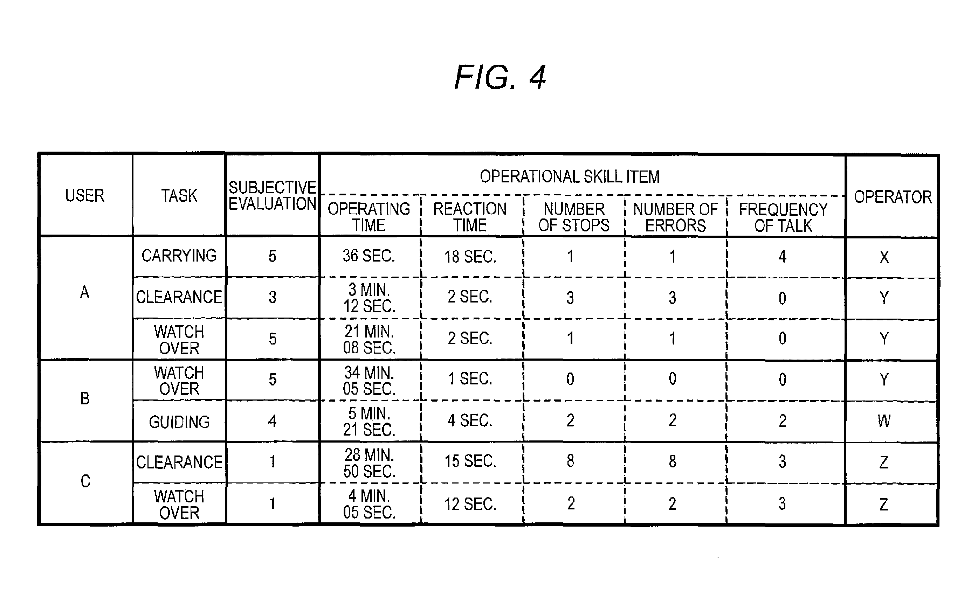

Fig. 4 shows examples of subjective evaluations from the user and operational skill parameters that are compiled. As described above, a subjective evaluation from the user is a score given subjectively by the user to each task performed by the robot. An operational skill parameter is the operation time of a service (remote control), the reaction time between an instruction from the user and reaction thereto, the number of times the robot stopped during remote control, the number of errors in remote control, or the frequency at which the user is talked to during operation, for example, measured each time a task is performed.

The relation between a subjective evaluation from the user and an operational skill parameter can be expressed by a general linear model, for example. In a case where the robot performs tasks #1 to #N, operational skill parameters of p operational skill items #1 to #p are calculated, subjective evaluations of a user on the tasks are represented by $y_1, ..., y_N$, and the operational skill parameters are represented by $x_1, ... , x_p$, the general linear model is expressed as in the following expression (1).

[Mathematical Formula 1]

$$Y = A_b X + E$$

$$Y = \begin{bmatrix} y_1 \\ \vdots \\ y_N \end{bmatrix}, X = \begin{bmatrix} x_1 \\ \vdots \\ x_p \end{bmatrix}, A_b = \begin{bmatrix} \alpha_1^T \\ \vdots \\ \alpha_p^T \end{bmatrix}, E = \begin{bmatrix} e_1 \\ \vdots \\ e_p \end{bmatrix} \quad \cdots (1)$$

In the expression (1), $A_b$ represents a weight on each operational skill parameter with respect to the subjective evaluations. In addition, E represents an error that expresses a part that cannot be expressed by the operational skill parameters alone.

When N = 1, that is, when a subjective evaluation is expressed by one item (task), the expression (1) is expressed as in the following expression (2).

[Mathematical Formula 2]

$$Y = \alpha_1 x_1 + \alpha_2 x_2 + \cdots + \alpha_p x_p + e \qquad \cdots (2)$$

In the expression (2), it is assumed that measurements of the subjective evaluation y and the operational skill parameters $x_1, ..., x_p$ from a user and a robot in the living space of the user are repeated. The weights $\alpha_i$ (i = 1 to p) on the respective operational skill parameters are estimated from the combinations of the measured $x_1, ..., x_p$ and subjective evaluation yby using the maximum likelihood estimation method, the least-squares method or the like.

If the operational skill parameters only include continuous values and categorical variables, the relation between the subjective evaluations and the operational skill parameters can be modeled by using multiple regression analysis.

Furthermore, if the operational skill parameters are on a nominal scale, on an ordinal scale, or in three or more categories, the relation between the subjective evaluations and the operational skill parameters can be modeled by using analysis of variance.

If the operational skill parameters are on a nominal scale and include a few continuous values, the relation between the subjective evaluations and the operational skill parameters by using analysis of covariance.

Which analysis method to use depends on what are used as the operational skill parameters.

It is then possible to know to which of the multiple operational skill items the user who purchased the robot places importance in the subjective evaluations from the estimated weights $\alpha_i$ (i = 1 to p) on the respective operational skill parameters. Furthermore, it is also possible to grasp which of the operational skill items each operator is good at or not good at by compiling the operational skill parameters respectively measured when one or more tasks are performed by remotely controlling the robot for one or more users. Thus, the service provider can match up operators according to the user's preference by appointing operators good at remote control of the robot on the operational skill item to which the user places importance.

In the analysis using the expressions (1) and (2), the weight $A_b$ is estimated on the assumption that the operational skill parameters X are independent of one another, that is, uncorrelated. The actual operational skill parameters, however, are supposed to be correlated. For example, when the operation time and the number of stops are taken as the operational skill parameters, the two variables are considered to be correlated. This is because the operation time will be longer as the number of stops increases. Subjective evaluations made by the user on two tasks are also considered to be correlated. Furthermore, a subjective evaluation made by the user on a task and an operational skill parameter measured when the robot performs the task are also considered to be correlated.

A structural equation model can be considered as a modeling method taking into consideration such correlation between operational skill parameters, correlation between subjective evaluations, and correlation between a subjective evaluation and an operational skill parameter. The structural equation model is given by the following expression (3).

[Mathematical Formula 3]

$$T = AT + U$$

$$T = \begin{bmatrix} X \\ Y \end{bmatrix}, U = \begin{bmatrix} D \\ E \end{bmatrix}, A = \begin{bmatrix} A_a & A_d \\ A_b & A_c \end{bmatrix} \qquad \cdots (3)$$

In the expression (3) , D represents an error with respect to a subjective evaluation Y, $A_a$ represents a coefficient matrix in which ij elements of coefficients $\alpha_{aij}$ expressing the degree to which an operational skill parameter $x_i$ can be determined from another operational skill parameter $x_j$ are arrayed, $A_c$ represents a coefficient matrix expressing the degree to which a subjective evaluation $y_i$ can be determined from another subjective evaluation $y_j$, and $A_d$ represents a coefficient matrix expressing the degree to which an operational skill parameter $x_i$ can be determined from a subjective evaluation $Y_j$.

In short, this can be said to be an extension of the general linear model (the expression (1)) including the coefficient matrix $A_a$ representing the relation between operational skill parameters and the coefficient matrix $A_c$ representing the relation between subjective evaluations. Thus, if the matrices $A_a$, $A_d$, $A_c$, and D are all zero matrices in the expression (3), the expression (3) will be as expressed by the following expression (4), which is the same as the expression (1).

[Mathematical Formula 4]

$$\begin{bmatrix} X \\ Y \end{bmatrix} = \begin{bmatrix} 0 & 0 \\ A_b & 0 \end{bmatrix} \begin{bmatrix} X \\ Y \end{bmatrix} + \begin{bmatrix} 0 \\ E \end{bmatrix} \qquad \cdots (4)$$

The general linear model can therefore be, in other words, a special case of a structural analysis model. Analysis of the relation between subjective evaluations made by the user and operational skill parameters of the operators using this structural analysis model allows the operational skill item to which the user places importance to be grasped more correctly. The structural equation model is excellent in clarifying the causal relationship between parameters and can also clarify the structure between parameters. Accordingly, the causal relationship including a change in an operator ID and time-series variation can be modeled in addition to the relation between subjective evaluations made by the user and operational skill parameters of the operators. In this case, the operator IDs and the time taken are also included in the matrices for analysis.

Furthermore, operational skill items at which each of the operators is good at or not good at can also be grasped. Then, the user and the operators can be matched up with each other by combining this with the result of analysis of subjective evaluations made by the user. As a result, a combination with an operator that can also appropriately and efficiently provide the service required by the user can be calculated.

The technologies disclosed herein can also have the following structures.

(1) An evaluation device including: a subjective evaluation acquiring unit configured to acquire a subjective evaluation made by a user on each task performed by a robot installed in the same space as the user; an operational skill parameter acquiring unit configured to acquire operational skill parameters on a plurality of operational skill items when the robot performs each task by being remotely controlled by an operator; and an analyzing unit configured to analyze a relation between the subjective evaluation acquired by the subjective evaluation acquiring unit and the operational skill parameters acquired by the operational skill parameter acquiring unit to determine an operational skill item to which the user places importance in subjective evaluation.

(2) The evaluation device according to (1), wherein analysis on a plurality of operators is conducted for one user, and the evaluation device further includes a selecting unit configured to select an operator having a high operational skill parameter on the operational skill item to which it is determined that the user places importance in subjective evaluation for the user.

(3) The evaluation device according to (1), wherein when the operational skill parameters are continuous values or categorical variables, the analyzing unit performs analysis by modeling the relation between the subjective evaluation and the operational skill parameters by using multiple regression analysis.

(4) The evaluation device according to (1), wherein when the operational skill parameters are on a nominal scale, on an ordinal scale, or in three or more categories, the analyzing unit performs analysis by modeling the relation between the subjective evaluation and the operational skill parameters by using analysis of variance.

(5) The evaluation device according to (1), wherein when the operational skill parameters are on a nominal scale and include a few continuous values, the analyzing unit performs analysis by modeling the relation between the subjective evaluation and the operational skill parameters by using analysis of covariance.

(6) The evaluation device according to (1), wherein the analyzing unit performs analysis by modeling the relation between the subjective evaluation and the operational skill parameters by using a structural equation taking correlation between two or more operational skills into consideration.

(7) The evaluation device according to (1), wherein the operational skill parameter acquiring unit acquires operation time of remote control performed by an operator as an operational skill parameter.

(8) The evaluation device according to (1), wherein the operational skill parameter acquiring unit acquires reaction time between an instruction from the user and a reaction of the robot as an operational skill parameter.

(9) The evaluation device according to (1), wherein the operational skill parameter acquiring unit acquires the number of times the robot stopped during remote control as an operational skill parameter.

(10) The evaluation device according to (1), wherein the operational skill parameter acquiring unit acquires the number of errors in remote control performed by an operator as an operational skill parameter.

(11) The evaluation device according to (1), wherein the operational skill parameter acquiring unit acquires a frequency at which the robot talks to the user during operation as an operational skill parameter.

(12) The evaluation device according to (1), wherein the operational skill parameter acquiring unit acquires at least two of reaction time between an instruction from the user and a reaction of the robot, the number of times the robot stopped during remote control, the number of errors in remote control performed by an operator, and a frequency at which the robot talks to the user during operation as the operational skill parameters.

(13) An evaluation method comprising: a subjective evaluation acquiring step of acquiring a subjective evaluation made by a user on each task performed by a robot installed in the same space as the user; an operational skill parameter acquiring step of acquiring operational skill parameters on a plurality of operational skill items when the robot performs each task by being remotely controlled by an operator; and an analyzing step of analyzing a relation between the subjective evaluation acquired in the subjective evaluation acquiring step and the operational skill parameters acquired in the operational skill parameter acquiring step to determine an operational skill item to which the user places importance in subjective evaluation.

(14) A service providing system comprising: a robot installed in the same space as a user; an operator terminal through which an operator remotely controls the robot; and a service provider server configured to match up a user with an operator on a basis of a subjective evaluation made by the user on each task performed by the robot and results of measuring operational skill parameters when the robot performs each task by being remotely controlled by the operator.

(15) A computer program described in a computer-readable format and causing a computer to function as: a subjective evaluation acquiring unit configured to acquire a subjective evaluation made by a user on each task performed by a robot installed in the same space as the user; an operational skill parameter acquiring unit configured to acquire operational skill parameters on a plurality of operational skill items when the robot performs each task by being remotely controlled by an operator; and an analyzing unit configured to analyze a relation between the subjective evaluation acquired by the subjective evaluation acquiring unit and the operational skill parameters acquired by the operational skill parameter acquiring unit to determine an operational skill item to which the user places importance in subjective evaluation.

INDUSTRIAL APPLICABILITY

The technologies disclosed herein have been described in detail above with reference to certain embodiments. It should be, however, obvious that those skilled in the art can modify or replace those embodiments with other embodiments without departing from the scope of the technologies disclosed herein.

While the embodiments applied to services through remote control of a robot that is operated in an everyday living space and perform nursing care and housework assistance have been describedherein, the scope to which the technologies disclosed herein are applied is not limited thereto. For example, the technologies disclosed herein can also be applied similarly to driving simulations and surgical operations, for example, and other services through remote control of a robot.

In short, the technologies have been disclosed in the form of examples, and the description of the specification should not be interpreted in a limited manner. The claims should be taken into account in understanding the subject matter of the technologies disclosed herein.

REFERENCE SIGNS LIST

31 Subjective evaluation input unit
32 Subjective evaluation acquiring unit
33 Operational skill parameter measuring unit
34 Operational skill parameter acquiring unit
35 Analyzing unit

**Claims**

1. An evaluation device comprising:

   a subjective evaluation acquiring unit configured to acquire a subjective evaluation made by a user on each task performed by a robot installed in the same space as the user;
   an operational skill parameter acquiring unit configured to acquire operational skill parameters on a plurality of operational skill items when the robot performs each task by being remotely controlled by an operator; and
   an analyzing unit configured to analyze a relation between the subjective evaluation acquired by the subjective evaluation acquiring unit and the operational skill parameters acquired by the operational skill parameter acquiring unit to determine an operational skill item to which the user places importance in subjective evaluation.

**2.** The evaluation device according to claim 1, wherein
analysis on a plurality of operators is conducted for one user, and
the evaluation device further comprises a selecting unit configured to select an operator having a high operational skill parameter on the operational skill item to which it is determined that the user places importance in subjective evaluation for the user.

**3.** The evaluation device according to claim 1, wherein when the operational skill parameters are continuous values or categorical variables, the analyzing unit performs analysis by modeling the relation between the subjective evaluation and the operational skill parameters by using multiple regression analysis.

**4.** The evaluation device according to claim 1, wherein when the operational skill parameters are on a nominal scale, on an ordinal scale, or in three or more categories, the analyzing unit performs analysis by modeling the relation between the subjective evaluation and the operational skill parameters by using analysis of variance.

**5.** The evaluation device according to claim 1, wherein when the operational skill parameters are on a nominal scale and include a few continuous values, the analyzing unit performs analysis by modeling the relation between the subjective evaluation and the operational skill parameters by using analysis of covariance.

**6.** The evaluation device according to claim 1, wherein the analyzing unit performs analysis by modeling the relation between the subjective evaluation and the operational skill parameters by using a structural equation taking correlation between two or more operational skills into consideration.

**7.** The evaluation device according to claim 1, wherein the operational skill parameter acquiring unit acquires operation time of remote control performed by an operator as an operational skill parameter.

**8.** The evaluation device according to claim 1, wherein the operational skill parameter acquiring unit acquires reaction time between an instruction from the user and a reaction of the robot as an operational skill parameter.

**9.** The evaluation device according to claim 1, wherein the operational skill parameter acquiring unit acquires the number of times the robot stopped during remote control as an operational skill parameter.

**10.** The evaluation device according to claim 1, wherein the operational skill parameter acquiring unit acquires the number of errors in remote control performed by an operator as an operational skill parameter.

**11.** The evaluation device according to claim 1, wherein the operational skill parameter acquiring unit acquires a frequency at which the robot talks to the user during operation as an operational skill parameter.

**12.** The evaluation device according to claim 1, wherein the operational skill parameter acquiring unit acquires at least two of reaction time between an instruction from the user and a reaction of the robot, the number of times the robot stopped during remote control, the number of errors in remote control performed by an operator, and a frequency at which the robot talks to the user during operation as the operational skill parameters.

**13.** An evaluation method comprising:

a subjective evaluation acquiring step of acquiring a subjective evaluation made by a user on each task performed by a robot installed in the same space as the user;
an operational skill parameter acquiring step of acquiring operational skill parameters on a plurality of operational skill items when the robot performs each task by being remotely controlled by an operator; and
an analyzing step of analyzing a relation between the subjective evaluation acquired in the subjective evaluation acquiring step and the operational skill parameters acquired in the operational skill parameter acquiring step to determine an operational skill item to which the user places importance in subjective evaluation.

**14.** A service providing system comprising:

a robot installed in the same space as a user;
an operator terminal through which an operator remotely controls the robot;
and a service provider server configured to match up a user with an operator on a basis of a subjective evaluation made by the user on each task performed by the robot and results of measuring operational skill parameters when the robot performs each task by being remotely controlled by the operator.

15. A computer program described in a computer-readable format and causing a computer to function as:

a subjective evaluation acquiring unit configured to acquire a subjective evaluation made by a user on each task performed by a robot installed in the same space as the user;
an operational skill parameter acquiring unit configured to acquire operational skill parameters on a plurality of operational skill items when the robot performs each task by being remotely controlled by an operator; and
an analyzing unit configured to analyze a relation between the subjective evaluation acquired by the subjective evaluation acquiring unit and the operational skill parameters acquired by the operational skill parameter acquiring unit to determine an operational skill item to which the user places importance in subjective evaluation.

# FIG. 1

# FIG. 2

S201 — INTRODUCE ROBOT BY USER

S202 — APPOINT OPERATOR BY SERVICE PROVIDER

S203 — OPERATE ROBOT IN LIVING SPACE OF USER BY OPERATOR

CALCULATE PARAMETER ON OPERATIONAL SKILL BY ROBOT

S204

S205 — EVALUATE SERVICE PROVIDED BY ROBOT BY USER

# FIG. 3

## FIG. 4

| USER | TASK | SUBJECTIVE EVALUATION | OPERATIONAL SKILL ITEM | | | | | OPERATOR |
|---|---|---|---|---|---|---|---|---|
| | | | OPERATING TIME | REACTION TIME | NUMBER OF STOPS | NUMBER OF ERRORS | FREQUENCY OF TALK | |
| A | CARRYING | 5 | 36 SEC. | 18 SEC. | 1 | 1 | 4 | X |
| A | CLEARANCE | 3 | 3 MIN. 12 SEC. | 2 SEC. | 3 | 3 | 0 | Y |
| A | WATCH OVER | 5 | 21 MIN. 08 SEC. | 2 SEC. | 1 | 1 | 0 | Y |
| B | WATCH OVER | 5 | 34 MIN. 05 SEC. | 1 SEC. | 0 | 0 | 0 | Y |
| B | GUIDING | 4 | 5 MIN. 21 SEC. | 4 SEC. | 2 | 2 | 2 | W |
| C | CLEARANCE | 1 | 28 MIN. 50 SEC. | 15 SEC. | 8 | 8 | 3 | Z |
| C | WATCH OVER | 1 | 4 MIN. 05 SEC. | 12 SEC. | 2 | 2 | 3 | Z |

EP 2 704 091 A1

**INTERNATIONAL SEARCH REPORT**

| International application No. |
| --- |
| PCT/JP2012/053958 |

A. CLASSIFICATION OF SUBJECT MATTER
*G06Q50/10*(2012.01)i, *G06Q50/22*(2012.01)i

According to International Patent Classification (IPC) or to both national classification and IPC

B. FIELDS SEARCHED

Minimum documentation searched (classification system followed by classification symbols)
G06Q50/10, G06Q50/22

Documentation searched other than minimum documentation to the extent that such documents are included in the fields searched
| Jitsuyo Shinan Koho | 1922-1996 | Jitsuyo Shinan Toroku Koho | 1996-2012 |
| Kokai Jitsuyo Shinan Koho | 1971-2012 | Toroku Jitsuyo Shinan Koho | 1994-2012 |

Electronic data base consulted during the international search (name of data base and, where practicable, search terms used)
JSTPlus(JDreamII), JMEDPlus(JDreamII), JST7580(JDreamII)

C. DOCUMENTS CONSIDERED TO BE RELEVANT

| Category* | Citation of document, with indication, where appropriate, of the relevant passages | Relevant to claim No. |
| --- | --- | --- |
| A | JP 2009-131914 A (Kabushiki Kaisha Advanced Telecommunications Research Institute International), 18 June 2009 (18.06.2009), entire text; all drawings (Family: none) | 1-15 |
| A | JP 2006-018768 A (Yugen Kaisha Ito Pharmacy), 19 January 2006 (19.01.2006), entire text; all drawings (Family: none) | 1-15 |
| A | JP 2001-250003 A (Yoichi SUGIMOTO), 14 September 2001 (14.09.2001), entire text; all drawings (Family: none) | 1-15 |

☐ Further documents are listed in the continuation of Box C.  ☐ See patent family annex.

| * | Special categories of cited documents: | "T" | later document published after the international filing date or priority date and not in conflict with the application but cited to understand the principle or theory underlying the invention |
| --- | --- | --- | --- |
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
| --- | --- |
| 01 March, 2012 (01.03.12) | 13 March, 2012 (13.03.12) |

| Name and mailing address of the ISA/ | Authorized officer |
| --- | --- |
| Japanese Patent Office | |
| Facsimile No. | Telephone No. |

Form PCT/ISA/210 (second sheet) (July 2009)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2010162687 A
- WO 2008140011 A